# EUROPÄISCHE PATENTSCHRIFT

(11) **EP 3 969 891 B1**
(45) Veröffentlichungstag und Bekanntmachung des Hinweises auf die Patenterteilung: **01.05.2024**
(21) Anmeldenummer: 20726734.5
(22) Anmeldetag: 11.05.2020
(51) Int. Cl.: G01M 13/04, G01N 33/20, G01N 27/904, G01N 27/9013

(54) **VERFAHREN UND VORRICHTUNG ZUR INDUKTIVEN PRÜFUNG VON METALLISCHEN WERKSTÜCKEN ZUR DETEKTION VON OBERFLÄCHENNAHEN ANOMALIEN**
METHOD AND DEVICE FOR INDUCTIVE TESTING OF METALLIC WORKPIECES TO DETECT ANOMALIES CLOSE TO THE SURFACE
PROCEDE ET DISPOSITIF DE CONTROLE INDUCTIF DE PIECES METALLIQUES POUR LA DETECTION D'ANOMALIES PROCHES DE LA SURFACE

(30) Priorität: 14.05.2019 DE 102019206989
(43) Veröffentlichungstag der Anmeldung: 23.03.2022
(73) Patentinhaber: thyssenkrupp rothe erde Germany GmbH, 44137 Dortmund (DE); thyssenkrupp AG, 45143 Essen (DE)
(72) Erfinder: NÄRMANN, Jürgen, 48317 Drensteinfurt (DE); LÜNEBURG, Bernd, 45481 Mülheim (DE); MOOK, Gerhard, 39104 Magdeburg (DE)
(74) Vertreter: thyssenkrupp Intellectual Property GmbH
(86) Internationale Anmeldenummer: PCT/EP2020/062962
(87) Internationale Veröffentlichungsnummer: WO 2020/229376

(56) Entgegenhaltungen:
- EP-A1- 0 529 354
- DE-A1-102013 209 808
- DE-A1-102016 200 690
- US-A1- 2005 206 374
- US-B1- 6 370 485

## Beschreibung

### Stand der Technik

Die Erfindung betrifft ein Verfahren zur induktiven Prüfung von metallischen Werkstücken zur Detektion von oberflächennahen Anomalien nach dem Oberbegriff des Anspruchs 1 und eine Vorrichtung nach dem Oberbegriff des Anspruchs 8.

Zur Qualitätsprüfung metallischer Werkstücke, insbesondere gehärteter Stahlwerkstücke, ist es üblich, die Werkstücke auf oberflächennahe Anomalien zu untersuchen. Derartige Anomalien sind lokale Gefügeveränderungen, die die mechanischen Eigenschaften des Werkstücks, wie z.B. die Betriebsfestigkeit, die Überrollfestigkeit und die Lebensdauer, negativ beeinflussen. Besonders relevante Anomalien werden auch als Ungänzen bezeichnet und umfassen insbesondere Risse, Einschlüsse, Poren und Lunker.

Ein aus dem Stand der Technik bekanntes Verfahren zur Prüfung von ferromagnetischen Werkstücken ist die sogenannte Magnetpulverrissprüfung. Bei dieser Prüfung wird ein flüssiges Prüfmittel, in dem pulverförmige, ferromagnetische Teilchen aufgeschwemmt sind, innerhalb einer Prüfanlage auf das Werkstück aufgebracht und das Werkstück wird magnetisiert. Oberflächennahe Anomalien rufen dabei lokale magnetische Streufelder hervor, an denen sich die pulverförmigen Teilchen ansammeln. Auf diese Weise können Risse und Poren bis zu einer gewissen Tiefe sichtbar gemacht werden. Nachteilig ist, dass aufgrund der korrosiven Wirkung der nötigen Benetzung die Prüfung nicht im Auslieferungszustand, sondern mit einem Aufmaß vor der Endbearbeitung in einer dafür vorgesehenen Prüfanlage stattfindet. Hierdurch ergibt sich eine Wartezeit vor Prüfung, die die Fertigstellung des Werkstücks um bis zu 24 Stunden verzögert. Ferner erlaubt das Verfahren nur eine direkte visuelle Auswertung durch den Mitarbeiter. Eine Speicherung der Ergebnisse ist nicht möglich.

Aus US 6 370 485 B1 ist ein Verfahren zur induktiven Prüfung von metallischen Werkstücken nach dem Oberbegriff des Anspruchs 1 und eine Vorrichtung nach dem Oberbegriff des Anspruchs 8 bekannt. Der bekannte Detektor zur Prüfung von Rohren ist vom Rotationstyp. Der Sensor wird während der Messung um die Achse des Rohres gedreht und gleichzeitig in axialer Richtung vorgeschoben. Der Detektor nimmt dadurch Datenpunkte in konstanten Abständen entlang einer definierten Spiralbahn auf. Wird das Messsignal als Funktion der Zeit ausgegeben, erhält man eine eindimensionale Wellenform in Abhängigkeit von der Zeit, die in ein Spiralkoordinatensystem und auch in ein ebenes rechtwinkliges Koordinatensystem umgerechnet werden kann, um anschließend eine Filterung im Ortsfrequenzraum vorzunehmen.

US 2005/0206374 A1 beschreibt eine Vorrichtung zum Prüfen eines metallischen Pfostens in einer einzigen Richtung auf Materialfehler. Die Vorrichtung hat eine Klemme mit mindestens einer Klemmbacke, deren Oberfläche der Kontur des Metallpfostens angepasst ist. Die angepasste Klemmbacke hat eine Vielzahl von Wirbelstromspulen und die Sonde hat mindestens einen Sensor, der so ausgelegt ist, dass er mindestens die Position oder die Bewegung der Sonde erfasst. Der Sensor kann beispielsweise ein Gummirad sein, welches eine kontrollierte Bewegung in axialer Richtung des Pfostens bewirkt.

DE 10 2016 200 690 A1 beschreibt ein Verfahren zur zerstörungsfreien Oberflächenprüfung eines Bauteils mit einem Wirbelstromprüfkopf, der ein Spulenarray mit zwei Reihen von Spulen umfasst. Für wenigstens ein Paar von Spulen, die in Längsrichtung unmittelbar benachbart und in unterschiedlichen Reihen angeordnet sind, wird ein Amplitudenwert der einen Spule zu einem zugeordneten Amplitudenwert der anderen Spule addiert und der Summenwert wird beim Vergleich der Amplitudenwerte zur Ermittlung einer relativen Fehlertiefe berücksichtigt.

DE 10 2013 209 808 A1 beschreibt einen induktiven Sensor mit drei mit ihren Flachseiten parallel zueinander gestapelt angeordneten und sich teilweise überlappenden Flachspulen zum Erfassen eines Material- und/oder Formparameters eines Metallgegenstandes.

EP 0 529 354 A1 beschreibt eine Vorrichtung zum Erkennen und Überwachen von Schäden an Laufbahnen oder angrenzenden Bereichen der Lagerringe eines Wälzlagers.

Aus DE 27 25 354 C2 ist ein Prüfkopf zur induktiven Oberflächenprüfung von metallischen Werkstücken mit Wirbelstromsonden in einem Trägerkörper bekannt, die durch jeweils eine ein- oder mehrlagige Spule mit Ferritkern gebildet und deren Achsen senkrecht zur Oberfläche des Werkstücks gerichtet sind. Jede Wirbelstromsonde ist Teil eines eigenen Schwingkreises, der mit einer Prüffrequenz betrieben wird. Wird der Prüfkopf über die Oberfläche eines metallischen Werkstücks geführt, treten in Abhängigkeit von darin vorhandenen Rissen Schwankungen im Gütefaktor der Schwingkreise auf, die es ermöglichen diese Risse zu detektieren. Nachteilig ist, dass sowohl größerflächige Gefügeveränderungen als auch lokal auftretende Anomalien Schwankungen des Gütefaktors hervorrufen, wobei der Verlauf des Messsignals von der bei der Messung verwendeten Vorschubgeschwindigkeit des Prüfkopfes stark abhängt. Für eine Auswertung des Messsignals sind daher umfangreiche Erfahrungswerte des Materialprüfers unerlässlich.

Aufgabe der Erfindung ist es, ein Verfahren und eine Vorrichtung zur induktiven Prüfung von metallischen Werkstücken anzugeben, mit denen eine berührungslose Bestimmung von Rohsignaldaten des Messkopfes zuordenbaren Positionsdaten unabhängig von der Vorschubgeschwindigkeit des Messkopfes ermöglicht wird.

Diese Aufgabe wird gelöst durch ein Verfahren zur induktiven Prüfung von metallischen Werkstücken zur Detektion von oberflächennahen Anomalien mit den Merkmalen des Anspruchs 1 und eine Vorrichtung mit den Merkmalen des Anspruchs 8.

Durch das erfindungsgemäße Verfahren werden die von der Wirbelstromsonde aufgenommenen Rohsignaldaten mit Positionsdaten zu einem ortsabhängigen Messsignal verknüpft. Das so erhaltene Messsignal enthält Informationen über die Qualität der Werkstückoberfläche entlang der zu prüfenden Bahn in Abhängigkeit von mindestens einer Ortsraumkoordinate. Der Einfluss von einer während des Prüfvorgangs verwendeten Vorschubgeschwindigkeit des Messkopfes und einer Abtastrate der Wirbelstromsonde, d.h. dem Zeitraum zwischen den Einzelmessungen, auf das auszuwertende Messsignal wird auf diese Weise eliminiert. Das Messsignal entspricht somit einer Kartierung der zu prüfenden Bahn.

Das Messsignal wird anschließend durch ein ortsfrequenzbasiertes Filter verarbeitet. Vorzugsweise werden bei der Weiterverarbeitung des Messsignals keine zeitbasierten Filter eingesetzt. Durch die Verarbeitung des Messsignals im Ortsfrequenzraum wird eine Auswertung des Messsignals ermöglicht, die den Ursachen der aufgenommenen Signalschwankungen angepasst ist. So weisen Gefügeveränderungen je nach deren Ursache unterschiedliche Ortsfrequenzsignaturen auf, die durch eine angepasste Wahl des Filters voneinander separiert werden können.

Die Erzeugung und/oder die Auswertung des Messsignals kann entweder während des Aufnehmens der Rohsignaldaten oder nachträglich auf Grundlage gespeicherter Rohsignaldaten erfolgen.

Das ortsfrequenzbasierte Filter umfasst vorzugsweise einen Hochpassfilter. Besonders bevorzugt weist das Hochpassfilter eine Grenzortsfrequenz auf, die im Bereich von 0,05 bis 1 Perioden pro mm liegt. Insbesondere kann die Grenzfrequenz des Hochpassfilters kontinuierlich oder in Stufen einstellbar sein, um die Gefügeveränderungen anhand ihrer Frequenzsignaturen voneinander separieren zu können. Größerflächige Gefügeveränderungen, wie sie beispielsweise durch geringfügige Schwankungen der Härteparameter oder Veränderungen im Basismaterial auftreten können, weisen eine vergleichsweise niedrige Ortsfrequenz auf. Rissbildungen, insbesondere solche, die beim Oberflächenhärten von Werkstücken auftreten können, oder auch Schleifbrand zeichnen sich durch eine höhere Ortsfrequenz aus. Bei dem Fehlerbild Schleifbrand handelt es sich um einen Fehler, der an der Oberfläche auftritt. Hervorgerufen wird dieser Fehler durch eine lokale thermische Beschädigung des Materials durch lokale Überhitzung, beispielsweise beim Schleifen der Oberfläche.

Das Hochpassfilter kann zudem durch ein Tiefpassfilter mit einer höheren Grenzfrequenz zu einem Bandpassfilter ergänzt sein, um einzelne Fehlerquellen getrennt voneinander untersuchen zu können.

Der Messkopf weist zumindest zwei in Bahnrichtung definiert beabstandet angeordnete Wirbelstromsonden auf, und die Positionsdaten werden aus einem Versatz der von diesen beiden Wirbelstromsonden erfassten Rohsignaldaten bestimmt. Daraus ergibt sich der Vorteil, dass die Positionsdaten berührungslos und ohne einen zusätzlichen Weg- oder Geschwindigkeitssensor bestimmt werden können. Aus dem Verhältnis des definierten Abstandes der Wirbelstromsonden zum festgestellten Versatz berechnet sich dann eine pro Messpunkt zurückgelegte Wegstrecke, die zur Bestimmung der Positionsdaten aufaddiert werden kann.

Wenn von einer annähernd konstanten Vorschubgeschwindigkeit des Messkopfes ausgegangen werden kann, kann eine einmalige Bestimmung des Versatzes ausreichend sein. Dieser kann beispielsweise über eine Korrelation der Rohsignaldaten beider Sensoren zueinander bestimmt werden.

Vorzugsweise wird der Versatz jedoch entlang der abgetasteten Bahn wiederholt bestimmt. Beispielsweise können hierfür die Rohsignaldaten für die gesamte Bahn in eine Mehrzahl von Abschnitten unterteilt werden, für die der jeweilige Versatz dann mittels Korrelation bestimmt wird.

Die zur Bestimmung der Positionsdaten verwendeten Wirbelstromsonden werden vorzugsweise mit einer höheren Abtastrate betrieben als weitere Wirbelstromsonden des Messkopfes. Dadurch kann eine erhöhte Ortsauflösung der Positionsdaten erreicht werden.

Vorzugsweise wird der Versatz der Rohsignaldaten auf Basis von erfassten Materialinhomogenitäten bestimmt. Materialinhomogenitäten sind Schwankungen der Materialeigenschaften ohne Relevanz für die mechanischen Eigenschaften, die in den Wirbelstromrohsignaldaten erkannt, aber nicht zur Bewertung verwendet werden.

Materialinhomogenitäten rufen wiedererkennbare Signalsignaturen in den Rohsignaldaten hervor, die über eine Mustererkennung identifiziert und einander zur Bestimmung des Versatzes zugeordnet werden können.

Das Verfahren kann auf Wälzlagerkomponenten als metallische Werkstücke angewendet werden. Insbesondere ist das Verfahren zur Prüfung von Wälzlagerringen geeignet. In einem bevorzugten Ausführungsbeispiel wird die oberflächengehärtete Wälzlagerlaufbahn eines Wälzlagerrings induktiv auf Anomalien geprüft.

Die Erfindung betrifft ferner eine Vorrichtung zur induktiven Prüfung von metallischen Werkstücken zur Detektion von oberflächennahen Anomalien, insbesondere Rissen, umfassend einen Messkopf, an dem zumindest eine Wirbelstromsonde angeordnet ist, zum Abtasten einer Oberfläche des Werkstücks entlang einer zu prüfenden Bahn, und eine Auswerteeinheit, die dazu ausgebildet ist, Rohsignaldaten der zumindest einen Wirbelstromsonde während des Abtastens der Werkstückoberfläche aufzunehmen. Die Auswerteeinheit ist ferner dazu ausgebildet, auf Grundlage von während des Abtastens aufgenommenen Messdaten eines Positions- oder Geschwindigkeitssensors und/oder auf Grundlage der Rohsignaldaten, Positionsdaten zu bestimmen, die den Rohsignaldaten zuordenbar sind. Die Auswerteeinheit ist weiterhin dazu ausgebildet, ein ortsabhängiges Messsignal durch Zuordnen der Positionsdaten zu den Rohsignaldaten zu erzeugen, wobei das Messsignal eine Funktion von zumindest einer Ortsraumkoordinate in Richtung der zu prüfenden Bahn ist, und ein gefiltertes Messsignal unter Anwendung eines ortsfrequenzbasierten Filters auf das Messsignal zu berechnen.

Der Messkopf weist zumindest zwei in Bahnrichtung definiert beabstandet angeordnete Wirbelstromsonden auf, und die Auswerteeinheit ist dazu ausgebildet, die Positionsdaten aus einem Versatz der von den beiden Wirbelstromsonden erfassten Rohsignaldaten zu bestimmen.

Weitere vorteilhafte Ausführungsformen sind der nachfolgenden Beschreibung und den Unteransprüchen zu entnehmen.

Die Erfindung wird nachstehend anhand der in den beigefügten Abbildungen dargestellten Ausführungsbeispiele näher erläutert.

### Kurzbeschreibung der Zeichnungen

- Fig. 1: zeigt schematisch in einer perspektivischen Ansicht ein erstes Ausführungsbeispiel der erfindungsgemäßen Vorrichtung bei der Durchführung des erfindungsgemäßen Verfahrens an einem Wälzlagerring eines Kegelrollenlagers,
- Fig.2: zeigt schematisch die Vorrichtung gemäß dem ersten Ausführungsbeispiel mit einer Querschnittsansicht des Wälzlagerrings,
- Fig.3: zeigt schematisch ein zweites Ausführungsbeispiel der erfindungsgemäßen Vorrichtung, die für die Prüfung der Laufbahn eines Kugellagerrings angepasst ist,
- Fig.4A: zeigt schematisch in einer Detaildarstellung ein Vergleichsbeispiel (nicht Teil der vorliegenden Erfindung) für den Aufbau eines Messkopfes mit mitgeführtem Reibrad,
- Fig. 4B: zeigt schematisch in einer Detaildarstellung ein Ausführungsbeispiel für den Aufbau eines Messkopfes mit zwei in Bahnrichtung definiert beabstandet angeordneten Wirbelstromsonden,
- Fig. 5: zeigt schematisch beispielhafte Rohsignaldaten der beiden in Bahnrichtung beabstandeten Wirbelstromsonden des Messkopfes gemäß Fig. 4B,
- Fig. 6: zeigt schematisch die aus den Rohsignaldaten gemäß Fig. 5 ermittelten Positionsdaten,
- Fig. 7: zeigt schematisch das aus den Rohsignaldaten gemäß Fig. 5 und den Positionsdaten gemäß Fig. 6 erzeugte Messsignal.

### Ausführungsformen der Erfindung

In den verschiedenen Figuren sind gleiche Teile stets mit den gleichen Bezugszeichen versehen und werden daher in der Regel auch jeweils nur einmal benannt bzw. erwähnt.

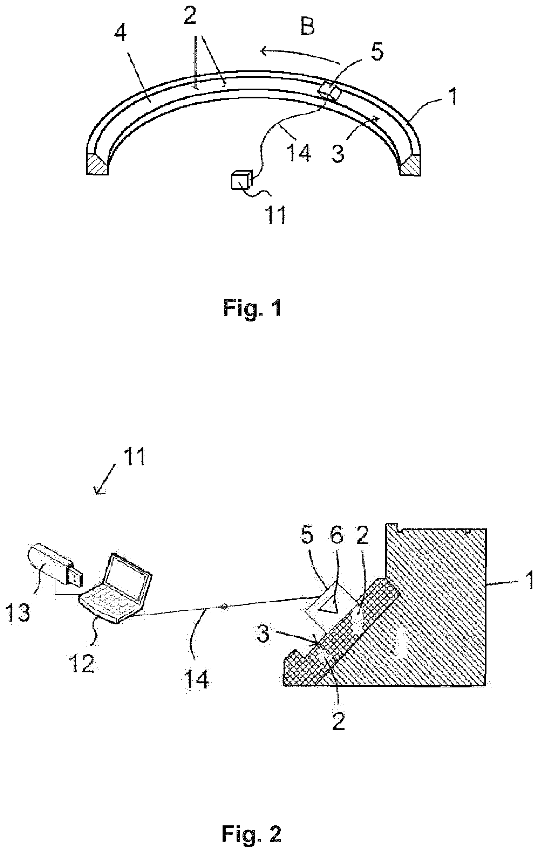

In **Fig. 1** ist eine Vorrichtung zur induktiven Prüfung von metallischen Werkstücken 1 zur Detektion von oberflächennahen Anomalien 2 dargestellt. Das Werkstück 1 ist in Fig. 1 beispielhaft ein Wälzlagerring. Die gehärtete Wälzlagerlaufbahn des Wälzlagerrings weist zumindest zwei Risse als Anomalien 2 auf.

Die Vorrichtung umfasst einen Messkopf 5, an dem zumindest eine Wirbelstromsonde 6 (vgl.

Fig. 2) angeordnet ist. Der Messkopf (5) weist zumindest zwei in Bahnrichtung (b) definiert beabstandet angeordnete Wirbelstromsonden (9) auf. Zum Abtasten einer Oberfläche 3 des Werkstücks 1 entlang einer zu prüfenden Bahn 4 kann der Messkopf 5 über die Werkstückoberfläche 3 in Bahnrichtung B geführt werden. Alternativ kann das Werkstück 1 an dem Messkopf 5 entlanggeführt werden. Die Vorrichtung umfasst ferner eine Auswerteeinheit 11, die mit dem Messkopf 5 über eine Signalleitung 14 verbunden ist.

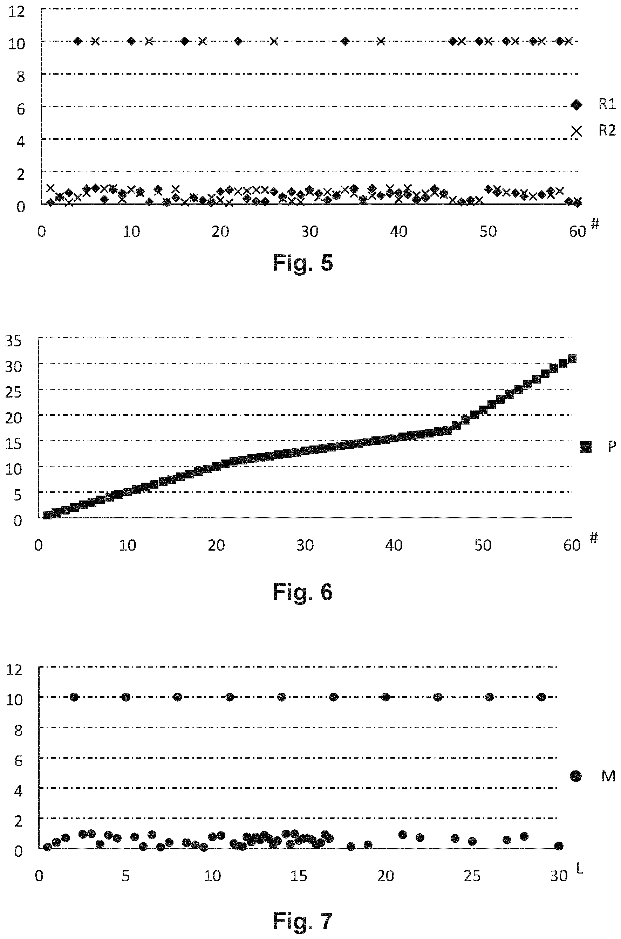

Die Vorrichtung ist dazu ausgebildet, das folgende Verfahren zur induktiven Prüfung des metallischen Werkstücks 1 zur Detektion der oberflächennahen Anomalien 2 auszuführen:
- Abtasten einer Oberfläche 3 des Werkstücks1 entlang der zu prüfenden Bahn 4 mit dem Messkopf 5, an dem die zumindest eine Wirbelstromsonde 6 angeordnet ist, und
- Aufnehmen von Rohsignaldaten R1, R2 (vgl. Fig. 5) der zumindest einen Wirbelstromsonde 6 während des Abtastens der Werkstückoberfläche 3,
- Bestimmen von Positionsdaten P (vgl. Fig. 6), die den Rohsignaldaten (R1, R2) zuordenbar sind, durch die Auswerteeinheit 11 auf Grundlage der Rohsignaldaten R1, R2,
- Erzeugen eines ortsabhängigen Messsignals M (vgl. Fig. 7) durch Zuordnen der Positionsdaten P zu den Rohsignaldaten R1, R2 durch die Auswerteeinheit 11, wobei das Messsignal M eine Funktion von zumindest einer Ortsraumkoordinate L in Richtung der zu prüfenden Bahn 4 ist,
- Berechnen eines gefilterten Messsignals durch die Auswerteeinheit 11 unter Anwendung eines ortsfrequenzbasierten Filters auf das Messsignal M,
- Bestimmung der Positionsdaten (P) auf Grundlage der Rohsignaldaten (R1, R2) aus einem Versatz der von den beiden Wirbelstromsonden (9) erfassten Rohsignaldaten (R1, R2).

**Fig. 2** zeigt das Ausführungsbeispiel gemäß Fig.1 in einer Schnittdarstellung des Wälzlagerrings. Durch das Abfahren der zu prüfenden Oberfläche können oberflächennahe Anomalien 2 in der gehärteten Wälzlagerlaufbahn erkannt werden. Dabei wird die Wirbelstromsonde 6 mit zumindest einer Prüffrequenz betrieben. Die Eindringtiefe in das Material des Werkstücks nimmt mit zunehmender Prüffrequenz der Wirbelstromsonde ab. Die Prüffrequenz der Wirbelstromsonden wird üblicherweise im Bereich von 2 kHz bis 20 kHz gewählt. Bei einer Prüffrequenz von 2 kHz sind Anomalien, wie beispielsweise Poren oder Materialeinschlüsse, bis zu einer Eindringtiefe von ca. 0,3 mm in dem metallischen Werkstück 1 detektierbar.

Als Auswerteeinheit kann beispielsweise ein tragbarer Computer 12 dienen, an den ein mobiler Datenträger 13, wie beispielsweise ein USB-Stick, mit einer Auswertesoftware angeschlossen ist. Die Auswertesoftware kann alternativ auf einem internen Datenträger des Computers 12 installiert sein. An den Computer 12 kann der Messkopf 5 beispielsweise über eine USB-Verbindung als Signalleitung 14 angeschlossen sein.

Die Ansteuerung der Wirbelstromsonden im Messkopf 5 sowie die Auswertung der Rohsignaldaten erfolgt durch den Computer 12. Die Wirbelstromsonden sind vorzugsweise als Halb-Transmission-Sensoren ausgebildet. Dabei wird ein im Computer 12 generiertes digitales Sendesignal über einen Digital-Analog-Wandler und vorzugsweise über eine analoge Anpassstufe an eine Sendespule der jeweiligen Wirbelstromsonde geleitet. Ein in einer zugeordneten Empfangsspule induziertes Empfangssignal wird vorzugsweise analog konditioniert und über einen Analog-Digital-Wandler dem Computer 12 als Rohsignaldaten zugeführt.

Vorzugsweise werden die gesamten Rohsignaldaten der Prüfung abgespeichert. Dadurch können die Rohsignaldaten nachträglich mit veränderbaren Auswerteparametern, wie beispielsweise angepassten Filtern, ausgewertet werden. Auch kann eine Datenbank von aufgenommenen Rohdaten nachträglich verwendet werden, um eine künstliche Intelligenz für die Erkennung von Anomalien zu trainieren.

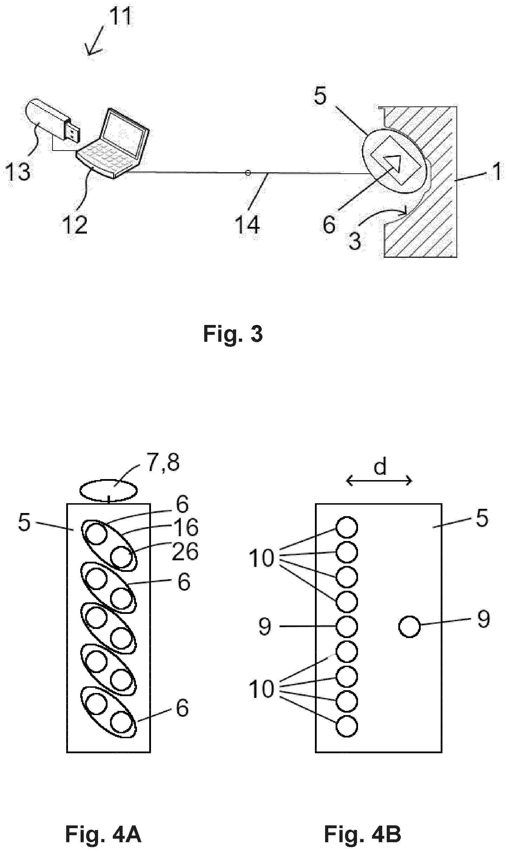

**Fig. 3** zeigt schematisch ein zweites Ausführungsbeispiel der erfindungsgemäßen Vorrichtung mit einem für die Prüfung einer Kugellagerlaufbahn angepassten Messkopf 5. Durch die Formanpassung des Messkopfes 5 kann ein konstanter Abstand zwischen der Wirbelstromsonde 6 und der zu prüfenden Werkstückoberfläche 3 sichergestellt werden.

Im Übrigen gelten die vorstehenden Ausführungen zum ersten Ausführungsbeispiel für das zweite Ausführungsbeispiel entsprechend.

**Fig. 4A und 4B** zeigen schematisch zwei Varianten des Aufbaus des Messkopfes 5, wobei Fig. 4A ein Vergleichsbeispiel darstellt und der Aufbau des Messkopfes nach Fig. 4B erfindungsgemäß in jedem der beiden vorgenannten Ausführungsbeispiele Verwendung finden kann.

Der Messkopf 5 gemäß Fig. 4A weist als Positions- und/oder Geschwindigkeitssensor 7 ein Reibrad 8 auf, das mit dem Messkopf 5 mitgeführt wird. Das Reibrad 8 gibt nach definierten zurückgelegten Weglängen einen Inkrementalimpuls aus, über deren Auswertung die Position des Messkopfes 5 im Verhältnis zu einem Startpunkt der Messung jederzeit abfragbar ist. Auf diese Weise sind den Rohsignaldaten zuordenbare Positionsdaten auf Basis der Messdaten des Reibrades bestimmbar.

Der Messkopf 5 umfasst ferner eine Mehrzahl von Wirbelstromsonden 6, beispielsweise fünf. Die Wirbelstromsoden 6 können vorzugsweise jeweils eine Sendespule 16 und eine Empfangsspule 26 aufweisen. Die Sende- 16 und die Empfangsspulen 26 sind vorzugsweise als sogenannte Schalenkerne, d.h. als Spule mit Ferritkern, ausgebildet. Die in der Empfangsspule 26 induzierte Spannung, die durch die von der Sendespule 16 in dem metallischen Werkstück erzeugten Wirbelströme hervorgerufen wird, wird demoduliert und als Messwert gespeichert. Die Spannung wird vorzugsweise als komplexwertiges Rohsignal mit Real- und Imaginärteil abgespeichert, um sowohl den Betrag als auch die Phasenverschiebung im Vergleich zur Sendespule 16 zu erfassen.

Die Mehrzahl der Wirbelstromsonden 6 sind quer zur Bahnrichtung angeordnet und bilden ein Wirbelstromsondenarray. Die einzelnen Wirbelstromsonden 6 werden vorzugsweise einzeln nacheinander elektronisch abgefragt. Diese Abfrage wird zyklisch über das gesamte Array verschoben. Dabei können wechselnde Zuordnungen von Sende- und Empfangsspulen zu Wirbelstromsonden 6 vorgesehen sein. Jede Spule kann somit als Sendespule 16 oder Empfangsspule 26 eingesetzt werden und mit jeder benachbarten Spule 16, 26 zeitweise eine abfragbare Wirbelstromsonde 6 bilden. Auf diese Weise kann die Ortsauflösung des Sondenarrays ohne zusätzliche Spulen 16, 26 erhöht werden. Die Anzahl der Wirbelstromsonden 6 des Arrays liegt vorzugsweise im Bereich von 5 bis 50.

Das Prinzip des Sondenarrays ist auf verschiedene Geometrien der abzutastenden Werkstückoberflächen anwendbar. Dabei können Sondenarrays sowohl für ebene Flächen als auch für außengekrümmte und innengekrümmte Flächen hergestellt werden. Um eine möglichst hohe Datenqualität zu erreichen, sollte für unterschiedliche Werkstückgeometrien jeweils ein angepasstes Sondenarray verwendet werden. Hierdurch kann ein im Wesentlichen konstanter Abstand zwischen den einzelnen Sonden und der Werkstückoberfläche erreicht werden.

In **Fig. 4B** ist der erfindungsgemäße Aufbau eines Messkopfes 5 mit zwei in Bahnrichtung definiert beabstandet angeordneten Wirbelstromsonden 9 gezeigt. Die Wirbelstromsonden 9 sind um den Abstand d zueinander beabstandet. Der Messkopf 5 weist zudem eine Mehrzahl weitere Wirbelstromsonden 10 auf, die in Bahnrichtung auf Höhe einer der beiden Wirbelstromsonden 9 angeordnet sind. Die Wirbelstromsonden 10 bilden ein sich quer zur Bahnrichtung erstreckendes Wirbelstromsondenarray. Die Wirbelstromsonden 9, 10 weisen bevorzugt, wie in Fig. 4A gezeigt, jeweils eine Sende- und eine Empfangsspule auf. Alternativ können die Wirbelstromsonden 9, 10 aber auch jeweils nur eine Spule aufweisen. In diesem Fall können die Rohsignaldaten aus einer Veränderung der Eigenfrequenz eines an diese Spule angeschlossenen Schwingkreises bestimmt werden.

Bevorzugt ist vorgesehen, dass die zur Bestimmung der Positionsdaten P verwendeten Wirbelstromsonden 9 mit einer höheren Abtastrate betrieben werden als die weiteren Wirbelstromsonden 10 des Messkopfes 5. Die höhere Abtastrate bewirkt ein verbessertes Signal - Rausch- Verhältnis, wodurch schwächere Signaländerungen, wie sie insbesondere durch Materialinhomogenitäten auftreten, besser aufgenommen und bewertet werden und zur Positionsbestimmung verwendet werden können.

Durch den in Fig. 4B gezeigten Aufbau des Messkopfes 5 ist es möglich die Positionsdaten P aus einem Versatz der von den beiden Wirbelstromsonden 9 erfassten Rohsignaldaten R1, R2 zu bestimmen. Dies hat den Vorteil, dass auf zusätzliche mechanische Komponenten, wie das in Fig. 4A vorgesehene Reibrad 8 zur Positionsbestimmung verzichtet werden kann, wodurch die Wartungshäufigkeit sinkt. Beim Abtasten der Werkstückoberfläche 3 braucht ferner nicht auf einen hinreichenden Kontakt des Reibrades 8 geachtet werden, so dass die Bedienung vereinfacht ist.

Bei den in Fig. 4A und 4B gezeigten Messköpfen liegt der Abstand der Wirbelstromsonden in Zeilenrichtung des Arrays bevorzugt im Bereich von 1-5 mm. Der Sondenabstand in Zeilenrichtung legt die Ortsauflösung des Messkopfes quer zur Bahnrichtung fest, d.h. den minimalen Abstand zweier Fehlstellen, bei dem diese getrennt voneinander detektierbar sind. Die Ortsauflösung in Bahnrichtung B wird durch das Verhältnis von Abtastrate (Anzahl aufgenommene Messwerte pro Zeiteinheit) zu Vorschubgeschwindigkeit bestimmt. Sowohl die Abtastrate als auch die Vorschubgeschwindigkeit sind wählbar.

Nachfolgend wird ein mögliches Verfahren zur Bestimmung der Positionsdaten und des Messsignals aus dem Versatz der Rohsignaldaten mit Bezug auf **Fig. 5 bis 7** beschrieben.

**Fig. 5** zeigt schematisch beispielhafte Rohsignaldaten R1, R2 der zwei in Bahnrichtung B definiert beabstandeten Wirbelstromsonden 9 des Messkopfes 5 gemäß Fig. 4B. Aufgetragen ist die Signalstärke S der durchnummerierten Datensätze. Auffälliges Charakteristikum der Rohsignaldaten R1 und R2 sind in unterschiedlichen Abständen auftretende Signalspitzen.

Zu jedem Messpunkt der Rohsignaldaten R1 existiert ein zugeordneter Messpunkt der Rohsignaldaten R2, der annähernd die gleiche Signalstärke aufweist, weil er am gleichen Ort der Bahn aufgenommen wurde. Der Versatz der beiden Signale R1, R2, d.h. die Anzahl Messpunkte zwischen einander zugeordneten Messpunkten kann variieren aufgrund von Schwankungen in der Vorschubgeschwindigkeit des Messkopfes und/oder in der Abtastrate. Der jeweils vorliegende Versatz entspricht immer einer durch den Messkopf 5 abgetasteten Strecke, die dem Abstand d der Wirbelstromsonden 9 entspricht. In Fig. 5 beträgt der Versatz in einem Anfangsabschnitt 2 Messpunkte, in einem mittleren Abschnitt 4 Messpunkte und in einem Endabschnitt 1 Messpunkt.

Der Versatz wird daher bevorzugt entlang der abgetasteten Bahn wiederholt bestimmt. Die Bestimmung des Versatzes kann dabei entweder für jeden Messpunkt, oder jeweils für aufeinanderfolgende Teilsequenzen von Messpunkten einzeln erfolgen. Bevorzugt weisen die Teilsequenzen dabei jeweils zumindest eine charakteristische Signatur einer Materialinhomogenität auf, die in den Rohsignaldaten R1, R2 wiedererkennbar ist.

Anschließend wird das Verhältnis von dem Abstand d zu dem jeweils vorliegenden Versatz für jeden Messpunkt bestimmt. Der Wert des Abstandes d ist vorzugsweise in der Auswerteeinheit 11 hinterlegt. Das resultierende Verhältnis definiert eine variierende, pro Messpunkt abgetastete Strecke. Diese pro Messpunkt zurückgelegten Strecken können zu Positionsdaten des Messkopfes 5 aufaddiert werden, um jedem Messpunkt die seit Beginn der Messung zurückgelegte Strecke zuzuordnen. **Fig. 6** zeigt die aus den Rohsignaldaten R1, R2 erzeugten Positionsdaten P, bei denen jeder Datensatz eine Ortsraumkoordinate L (in einer willkürlichen Einheit) in Richtung B der zu prüfenden Bahn enthält.

Wie in **Fig. 7** gezeigt, können die so erzeugten Positionsdaten P dann den Rohsignaldaten R1 zugeordnet werden, um ein Messsignal M zu erzeugen, das eine Funktion von zumindest einer Ortsraumkoordinate L in Richtung der zu prüfenden Bahn 4 ist. Erst in dem so erzeugten Messsignal M ist erkennbar, dass die Signalspitzen eine regelmäßige Fehlerstruktur mit einer definierten Ortsfrequenz bilden. Auf Basis des Messsignals M können daher bestimmte Fehlerstrukturen unter Anwendung eines ortsfrequenzbasierten Filters auf das Messsignal M extrahiert und in dem resultierenden gefilterten Messsignal mit einer verbesserten Genauigkeit detektiert werden.

Bevorzugt wird hierfür ein Hochpassfilter als ortsfrequenzbasiertes Filter eingesetzt. Das Hochpassfilter weist vorzugsweise eine Grenzortsfrequenz auf, die im Bereich von 1-1/20 Perioden/mm liegt. Insbesondere kann die Grenzortsfrequenz, beispielsweise über eine Software der Auswerteeinheit 11, einstellbar sein.

Durch die Umrechnung der Rohsignaldaten R1, R2 wird ein Messsignal M erzeugt, das unabhängig von der Vorschubgeschwindigkeit des Messkopfes 5 während der Messung ist. Dadurch ist es möglich die Prüfung nahezu ortsunabhängig durchzuführen, sodass der Messkopf 5 von Hand über die Werkstückoberfläche 3 geführt werden kann. Alternativ kann der Messkopf 5 auch an ein sich daran vorbei bewegendes Werkstück gehalten werden. Prüfanlagen, die eine Steuerung der Vorschubgeschwindigkeit auf einen konstanten Wert sicherstellen, sind nicht erforderlich. Die Prüfung kann daher insbesondere im Endzustand der Werkstückbearbeitung erfolgen.

Das zuvor beschriebene erfindungsgemäße Verfahren und die erfindungsgemäße Vorrichtung eignen sich insbesondere zur induktiven Prüfung von Wälzlagerlaufbahnen in Wälzlagerringen. Die Qualität der gehärteten Wälzlagerlaufbahn ist entscheidend für die Belastbarkeit und die Zuverlässigkeit der Lager. Oberflächennahe Anomalien können dabei insbesondere beim Oberflächenhärten der Laufbahn auftreten, weshalb das Verfahren und die Vorrichtung vorzugsweise zur Prüfung von induktions-, einsatz- oder flammgehärteten Wälzlagerlaufbahnen eingesetzt werden kann.

### Bezugszeichenliste

- 1: Werkstück
- 2: Anomalie
- 3: Werkstückoberfläche
- 4: Bahn
- 5: Messkopf
- 6: Wirbelstromsonde
- 7: Positions- und/oder Geschwindigkeitssensor
- 8: Reibrad
- 9: Wirbelstromsonde
- 10: Wirbelstromsonde
- 11: Auswerteeinheit
- 12: Computer
- 13: mobiler Datenträger
- 14: Signalleitung
- 16: Sendespule
- 26: Empfangsspule

- B: Bahnrichtung
- L: Ortsraumkoordinate
- M: Messsignal
- P: Positionsdaten
- R1, R2: Rohsignaldaten
- S: Signalstärke

## Patentansprüche

1. Verfahren zur induktiven Prüfung von metallischen Werkstücken (1) zur Detektion von oberflächennahen Anomalien (2), insbesondere Rissen, umfassend die folgenden Schritte:
- Abtasten einer Oberfläche (3) des Werkstücks (1) entlang einer zu prüfenden Bahn (4) mit einem Messkopf (5), an dem zumindest eine Wirbelstromsonde (6, 9, 10) angeordnet ist, und
- Aufnehmen von Rohsignaldaten (R1, R2) der zumindest einen Wirbelstromsonde (6, 9, 10) während des Abtastens der Werkstückoberfläche (3),
- Bestimmen von Positionsdaten (P), die den Rohsignaldaten (R1, R2) zuordenbar sind,
- Erzeugen eines ortsabhängigen Messsignals (M) durch Zuordnen der Positionsdaten (P) zu den Rohsignaldaten (R1, R2), wobei das Messsignal (M) eine Funktion von zumindest einer Ortsraumkoordinate (L) in Richtung der zu prüfenden Bahn (4) ist,
- Berechnen eines gefilterten Messsignals unter Anwendung eines ortsfrequenzbasierten Filters auf das Messsignal (M),
**dadurch gekennzeichnet dass**
der Messkopf (5) zumindest zwei in Bahnrichtung (B) definiert beabstandet angeordnete Wirbelstromsonden (9) aufweist, und die Positionsdaten (P) auf Grundlage der Rohsignaldaten (R1, R2) aus einem Versatz der von diesen beiden Wirbelstromsonden (9) erfassten Rohsignaldaten (R1, R2) bestimmt werden.

2. Verfahren nach Anspruch 1, **dadurch gekennzeichnet, dass** die zur Bestimmung der Positionsdaten (P) verwendeten Wirbelstromsonden (9) mit einer höheren Abtastrate betrieben werden als weitere Wirbelstromsonden (10) des Messkopfes (5).

3. Verfahren nach Anspruch 1 oder 2, **dadurch gekennzeichnet, dass** der Versatz entlang der abgetasteten Bahn (4) wiederholt bestimmt wird.

4. Verfahren nach einem der Ansprüche 1 bis 3, **dadurch gekennzeichnet, dass** der Versatz der Rohsignaldaten (R1, R2) auf Basis von erfassten Materialinhomogenitäten bestimmt wird.

5. Verfahren nach einem der Ansprüche 1 bis 4, **dadurch gekennzeichnet, dass** das ortsfrequenzbasierte Filter ein Hochpassfilter umfasst.

6. Verfahren nach Anspruch 5, **dadurch gekennzeichnet, dass** das Hochpassfilter eine Grenzortsfrequenz aufweist, die im Bereich von 0.05 bis 1 Periode/mm liegt.

7. Verfahren nach einem der Ansprüche 1 bis 6, **dadurch gekennzeichnet, dass** das metallische Werkstück (1) eine Wälzlagerkomponente, insbesondere ein Wälzlagerring ist.

8. Vorrichtung zur induktiven Prüfung von metallischen Werkstücken (1) zur Detektion von oberflächennahen Anomalien (2), insbesondere Rissen, umfassend
- einen Messkopf (5), an dem zumindest eine Wirbelstromsonde (6, 9, 10) angeordnet ist, zum Abtasten einer Oberfläche (3) des Werkstücks (1) entlang einer zu prüfenden Bahn (4),
- eine Auswerteeinheit (11), die dazu ausgebildet ist, Rohsignaldaten (R1, R2) der zumindest einen Wirbelstromsonde (6, 9, 10) während des Abtastens der Werkstückoberfläche (3) aufzunehmen,
wobei die Auswerteeinheit (11) ferner dazu ausgebildet ist, Positionsdaten (P) zu bestimmen, die den Rohsignaldaten (R1, R2) zuordenbar sind, ein ortsabhängiges Messsignal (M) durch Zuordnen der Positionsdaten (P) zu den Rohsignaldaten (R1, R2) zu erzeugen, wobei das Messsignal (M) eine Funktion von zumindest einer Ortsraumkoordinate (L) in Richtung der zu prüfenden Bahn (4) ist, und ein gefiltertes Messsignal unter Anwendung eines ortsfrequenzbasierten Filters auf das Messsignal (M) zu berechnen, **dadurch gekennzeichnet, dass** der Messkopf (5) zumindest zwei in Bahnrichtung (B) definiert beabstandet angeordnete Wirbelstromsonden (9) aufweist, und die Auswerteeinheit (11) dazu ausgebildet ist, die Positionsdaten (P) auf Grundlage der Rohsignaldaten (R1, R2) aus einem Versatz der von den beiden Wirbelstromsonden (9) erfassten Rohsignaldaten (R1, R2) zu bestimmen.

## Claims

1. A method for inductive testing of metallic workpieces (1) for the detection of anomalies (2) near the surface, in particular cracks, comprising the following steps:
- Scanning a surface (3) of the workpiece (1) along a path (4) to be tested with a measuring head (5) on which at least one eddy current probe (6, 9, 10) is arranged, and
- Recording of raw signal data (R1, R2) of the at least one eddy current probe (6, 9, 10) during the scanning of the workpiece surface (3),
- Determining of position data (P) that can be assigned to the raw signal data (R1, R2),
- Generating a position-dependent measurement signal (M) by assigning the position data (P) to the raw signal data (R1, R2), the measurement signal (M) being a function of at least one spatial coordinate (L) in the direction of the path (4) to be tested,
- Calculating a filtered measurement signal by applying a spatial frequency-based filter to the measurement signal (M),
**characterized in that**
the measuring head (5) has at least two eddy current probes (9) arranged at a defined distance in the path direction (B), and the position data (P) are determined on the basis of the raw signal data (R1, R2) from an offset of the raw signal data (R1, R2) recorded by these two eddy current probes (9).

2. The method according to claim 1, **characterized in that** the eddy current probes (9) used to determine the position data (P) are operated at a higher sampling rate than other eddy current probes (10) of the measuring head (5).

3. The method according to claim 1 or 2, **characterized in that** the offset along the scanned path (4) is determined repeatedly.

4. The method according to one of claims 1 to 3, **characterized in that** the offset of the raw signal data (R1, R2) is determined on the basis of detected material inhomogeneities.

5. The method according to any one of claims 1 to 4, **characterized in that** the spatial frequency-based filter comprises a high-pass filter.

6. The method according to claim 5, **characterized in that** the high-pass filter has a cutoff frequency which is in the range from 0.05 to 1 periods/mm.

7. The method according to one of claims 1 to 6, **characterized in that** the metallic workpiece (1) is a component of a rolling bearing, in particular a rolling bearing ring.

8. A device for inductive testing of metallic workpieces (1) for the detection of anomalies (2) near the surface, in particular cracks, comprising
- a measuring head (5), on which at least one eddy current probe (6, 9, 10) is arranged, for scanning a surface (3) of the workpiece (1) along a path (4) to be tested,
- an evaluation unit (11) which is designed to record raw signal data (R1, R2) of the at least one eddy current probe (6, 9, 10) during the scanning of the workpiece surface (3),
wherein the evaluation unit (11) is further designed to determine position data (P) which can be assigned to the raw signal data (R1, R2), to generate a location-dependent measurement signal (M) by assigning the position data (P) to the raw signal data (R1, R2), wherein the measurement signal (M) is a function of at least one spatial coordinate (L) in the direction of the path (4) to be tested, and to calculate a filtered measurement signal by applying a spatial frequency-based filter to the measurement signal (M), **characterized in that** the measuring head (5) has at least two eddy current probes (9) arranged at a defined distance in the path direction (B), and the evaluation unit (11) is designed to determine the position data (P) on the basis of the raw signal data (R1, R2) from an offset of the raw signal data (R1, R2) recorded by the two eddy current probes (9).

## Revendications

1. Procédé de contrôle inductif de pièces métalliques (1) pour la détection d'anomalies (2) proches de la surface, en particulier de fissures, comprenant les étapes suivantes:
- balayer une surface (3) de la pièce (1) le long d'une trajectoire (4) à contrôler avec une tête de mesure (5) sur laquelle est disposée au moins une sonde à courants de Foucault (6, 9, 10), et
- enregistrer des données de signal brutes (R1, R2) de la au moins une sonde à courants de Foucault (6, 9, 10) pendant le balayage de la surface (3) de la pièce,
- déterminer des données de position (P) qui peuvent être attribuées aux données de signal brutes (R1, R2),
- générer un signal de mesure (M) dépendant de l'emplacement en associant les données de position (P) aux données de signal brutes (R1, R2), le signal de mesure (M) étant une fonction d'au moins une coordonnée d'espace local (L) dans la direction de la trajectoire (4) à contrôler,
- calculer un signal de mesure filtré en appliquant un filtre basé sur la fréquence locale au signal de mesure (M),
**caractérisé en ce que**
la tête de mesure (5) présente au moins deux sondes à courants de Foucault (9) disposées à une distance définie dans la direction de la trajectoire (B), et les données de position (P) sont déterminées sur la base des données de signal brutes (R1, R2) à partir d'un décalage des données de signal brutes (R1, R2) saisies par ces deux sondes à courants de Foucault (9).

2. Procédé selon la revendication 1, **caractérisé en ce que** les sondes à courants de Foucault (9) utilisées pour déterminer les données de position (P) sont exploitées avec un taux d'échantillonnage plus élevé que d'autres sondes à courants de Foucault (10) de la tête de mesure (5).

3. Procédé selon la revendication 1 ou 2, **caractérisé en ce que** le décalage est déterminé de manière répétée le long de la trajectoire (4) balayée.

4. Procédé selon l'une des revendications 1 à 3, **caractérisé en ce que** le décalage des données de signal brutes (R1, R2) est déterminé sur la base des inhomogénéités de matériau détectées.

5. Procédé selon l'une quelconque des revendications 1 à 4, **caractérisé en ce que** le filtre basé sur la fréquence locale comprend un filtre passe-haut.

6. Procédé selon la revendication 5, **caractérisé en ce que** le filtre passe-haut présente une fréquence locale de coupure qui se situe dans la plage de 0,05 à 1 période/mm.

7. Procédé selon l'une des revendications 1 à 6, **caractérisé en ce que** la pièce métallique (1) est un composant de palier à roulement, en particulier une bague de palier à roulement.

8. Dispositif de contrôle inductif de pièces métalliques (1) pour la détection d'anomalies (2) proches de la surface, en particulier de fissures, comprenant
- une tête de mesure (5), sur laquelle est disposée au moins une sonde à courants de Foucault (6, 9, 10), pour balayer une surface (3) de la pièce (1) le long d'une trajectoire (4) à contrôler,
- une unité d'évaluation (11) qui est conçue pour enregistrer des données de signal brutes (R1, R2) de la au moins une sonde à courants de Foucault (6, 9, 10) pendant le balayage de la surface (3) de la pièce,
l'unité d'évaluation (11) étant en outre conçue pour déterminer des données de position (P) qui peuvent être associées aux données de signal brut (R1, R2), pour générer un signal de mesure (M) dépendant de la position en associant les données de position (P) aux données de signal brut (R1, R2), le signal de mesure (M) étant une fonction d'au moins une coordonnée d'espace local (L) dans la direction de la trajectoire (4) à contrôler, et à calculer un signal de mesure filtré en utilisant un filtre basé sur la fréquence locale sur le signal de mesure (M), **caractérisé en ce que** la tête de mesure (5) présente au moins deux sondes à courants de Foucault (9) disposées à une distance définie dans la direction de la trajectoire (B), et l'unité d'évaluation (11) est conçue pour déterminer les données de position (P) sur la base des données de signal brutes (R1, R2) à partir d'un décalage des données de signal brutes (R1, R2) saisies par les deux sondes à courants de Foucault (9).
